# FASCICULE DE BREVET EUROPEEN

(11) **EP 1 937 249 B1**
(45) Date de publication et mention de la délivrance du brevet: **25.04.2012**
(21) Numéro de dépôt: 06793080.0
(22) Date de dépôt: 30.08.2006
(51) Int. Cl.: A61K 31/34, A61K 8/49, A61Q 19/02

(54) **UTILISATION D'AU MOINS UN 2-ALKYLE FURANE, A TITRE DE PRINCIPE ACTIF DEPIGMENTANT OU ECLAIRCISSANT**
VERWENDUNG VON MINDESTENS EINEM 2-ALKYL-FURAN ALS WIRKSTOFF ZUR DEPIGMENTIERUNG ODER AUFHELLUNG
USE OF AT LEAST ONE 2-ALKYL FURAN, AS DEPIGMENTING OR LIGHTENING ACTIVE PRINCIPLE

(30) Priorité: 30.08.2005 FR 0508866
(43) Date de publication de la demande: 02.07.2008
(73) Titulaire: Laboratoires Expanscience, 92400 Courbevoie (FR)
(72) Inventeur: MSIKA, Philippe, F-78000 Versailles (FR); PICCARDI, Nathalie, F-21310 Arceau (FR)
(74) Mandataire: Warcoin, Jacques
(86) Numéro de dépôt international: PCT/EP2006/065836
(87) Numéro de publication internationale: WO 2007/025994

(56) Documents cités:
- EP-A1- 0 775 480
- EP-B1- 1 213 975
- EP-B1- 1 530 430
- WO-A-99/43298
- FR-A- 2 822 068
- FR-A- 2 870 742
- FR-A1- 2 678 632
- FR-A1- 2 798 667
- MAHÉ A ET AL: "Skin diseases associated with the cosmetic use of bleaching products in women from Dakar, Senegal." THE BRITISH JOURNAL OF DERMATOLOGY. MAR 2003, vol. 148, no. 3, mars 2003 (2003-03), pages 493-500, XP002386273 ISSN: 0007-0963

## Description

La présente invention se rapporte à l'utilisation d'une composition cosmétique à action dépigmentante ou éclaircissante, comprenant, à titre de principe actif, au moins un 2-alkyle furanne.

Les 2-alkyle furannes sont des furannes monosubstitués en position 2, qui peuvent notamment être extraits de l'avocat. Leur utilisation a déjà été décrite pour le traitement du diabète (demande internationale n°PCT/FR/2005/001310), de la cellulite (demande internationale n°PCT/FR2005/01311) et de l'obésité (demande internationale n°PCT/FR2005/01312).

La couleur de la peau est due à plusieurs substances : l'hémoglobine des vaisseaux, les caroténoïdes du derme et, surtout, la mélanine de l'épiderme. Cette mélanine est produite par les mélanocytes de la couche basale, sous l'action de la tyrosinase, du cuivre et de l'oxygène.

La mélanine de la peau est formée par une association complexe d'eumélanine et de phéomélanine.

Leurs biosynthèses sont communes jusqu'à la dopaquinone (double oxydation de la tyrosine par la tyrosinase, enzyme cupro-protéique). Leur chemin ensuite diverge.

L'eumélanine brune est un polymère d'indole-5-6-quinone tandis que la phéomélanine responsable de la couleur rousse est un composé contenant près de 10% de soufre et de structure polymère de la cystéinyl dopa.

D'autres enzymes que la tyrosinase participent à la genèse et au contrôle des mélanines : la dopachrome oxydo-réductase, l' α-glutamyl transpepsidase, le système glutathion (réductase-péroxydase), la dopachrome tautomérase.

Sous l'effet de stimulations exogènes ou endogènes, il peut apparaître des modifications de la teinte de la peau : ce sont les dyschromies (hyperchromie et hypochromie).

Ces modifications peuvent siéger dans l'épiderme ou dans le derme et être dues à une variation de la quantité de mélanine ou du nombre de mélanocytes.

Les hyperchromies sont des accumulations de pigments mélaniques, de caroténoïdes ou de pigments exogènes. Parmi les hyperchromies, nous pouvons citer le mélasma, qui est défini comme une hypermélanose acquise du visage qui peut s'observer dans les deux sexes et dans toutes les races. Le mélasma apparaît plus fréquemment chez les femmes utilisant une contraception orale ou pendant la grossesse (masque de grossesse, chloasma).

Le masque de grossesse, ou chloasma, apparaît chez les femmes qui ont un taux d'hormones féminines important et dont la peau est exposée au soleil. Il touche ainsi principalement les femmes enceintes ou les femmes prenant une pilule contraceptive. Il prend la forme de plaques pigmentées de couleur marron, souvent symétriques, de forme plus ou moins régulière.

Le vieillissement cutané se caractérise également par l'apparition de tâches pigmentaires. On parle alors de lentigo solaires pour les zones les plus fréquemment photo-exposées (visage, mains, décolleté), et de lentigo séniles, tâches pigmentaires assez larges, qui apparaissent chez le sujet âgé au niveau des mains, du visage et des bras.

Les agents dépigmentants ou blanchissants du teint sont des composés chimiques capables d'agir à l'échelon tissulaire, cellulaire, ou subcellulaire. Ils agissent sur la formation, le transport, la couleur de la mélanine elle-même ou sur l'existence de mélanocyte (mélanocytotoxicité).

D'autre part, il est nécessaire de mettre en évidence et de supprimer le facteur induisant l'hyperpigmentation avant de la traiter (U.V., parfum, oestroprogestatif) et de conseiller une protection solaire type protection maximale tout au long du suivi médical.

Enfin, il est possible d'éliminer les couches superficielles de cornéocytes contenant de la mélanine, et ainsi réaliser une dépigmentation physique de surface, traitement qui favorise par ailleurs la pénétration des agents dépigmentants.

Les motivations qui poussent à décolorer la peau peuvent être diverses. L'éclaircissement franc du teint est recherché en Afrique Noire avec des solutions traditionnelles ou chimiques qui présentent des effets secondaires néfastes importants sur l'aspect et la structure de la peau. La pâleur ou la blancheur du visage asiatique est obtenue avec des molécules agissant avec le moins de toxicité possible (l'arbutine, l'acide kojique, l'acide ascorbique).

Le traitement des taches d'hyperpigmentation des sujets blancs fait appel à des molécules diverses dont la principale, l'hydroquinone, fait l'objet d'une surveillance accrue et dont la dose maximale en cosmétique est de 2%.

Il existe donc un besoin pour des compositions présentant une activité dépigmentante ou éclaircissante et qui soient bien tolérées par la peau.

C'est pourquoi la présente invention a pour objet l'utilisation d'une fraction furanique purifiée de l'insaponifiable d'avocat comprenant des 2-alkyle furannes à titre de principe actif cosmétique dépigmentant ou éclaircissant du teint, dans une composition cosmétique.

L'invention concerne l'utilisation d'une fraction furanique purifiée de l'insaponifiable d'avocat comprenant des 2-alkyle furannes pour la fabrication d'une composition cosmétique dépigmentante. La composition cosmétique dépigmentante est avantageusement destinée à réduire et/ou supprimer et/ou prévenir les taches de pigmentation ou à éclaircir la peau naturellement pigmentée.

Dans le cadre de la présente invention, on entend par l'expression «2-alkyle furanne » les alkyles furannes, monosubstitués en position 2, répondant à la formule générale (I) 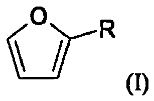 dans laquelle R représente un radical alkyle en C₁₀-C₂₂, encore plus avantageusement en C₁₂-C₂₀, encore plus avantageusement en C₁₃-C₁₇, un radical alcényle en C₁₀-C₂₂, encore plus avantageusement en C₁₂-C₂₀, encore plus avantageusement en C₁₃-C₁₇ ou un radical alcynyle en C₁₀-C₂₂, encore plus avantageusement en C₁₂-C₂₀, encore plus avantageusement en C₁₃-C₁₇, lesdits radicaux alkyles, alcényles et alcynyles pouvant être substitués par un ou plusieurs halogène(s) et/ou par un ou plusieurs fonctions choisies dans le groupe constitué par les fonctions époxyde, hydroxyle (-OH), thiol (-SH), éther (-OR₁), amine primaire (-NH₂), amine secondaire (-NHR₁), amine tertiaire (-NR₁R₂), aldéhyde (-CHO), cétone (-COR₁), acétyle (-O-CO-R₁), avec R₁ et R₂ représentant, indépendamment l'un de l'autre, un atome d'hydrogène, un radical alkyle en C₁-C₃₅, avantageusement en C₁₀-C₂₂ encore plus avantageusement en C₁₂-C₂₀, encore plus avantageusement en C₁₃-C₁₇ ou un radical alcényle en C₁-C₃₅, avantageusement en C₁₀-C₂₂ encore plus avantageusement en C₁₂-C₂₀, encore plus avantageusement en C₁₃-C₁₇.

Dans le cadre de la présente invention, le terme « alkyle » désigne un résidu hydrocarboné à chaîne droite ou ramifiée insaturé. Le terme « alcényle » désigne un résidu hydrocarboné à chaîne droite ou ramifiée comprenant des insaturations oléfiniques (double(s) liaison(s)). Le terme « alcynyle » désigne un résidu hydrocarboné à chaîne droite ou ramifiée comprenant au moins une triple liaison et éventuellement des insaturations oléfiniques.

Selon une variante avantageuse de l'invention, lesdits alkyles furannes sont des 2-alkyles furannes naturels, présents dans l'insaponifiable d'avocat furanique, répondant à la formule générale (I) dans laquelle R représente un radical choisi dans le groupe constitué par les radicaux suivants (*-R) :

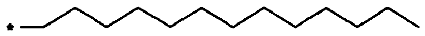

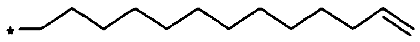

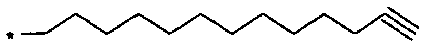

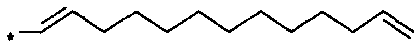

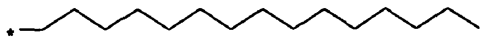

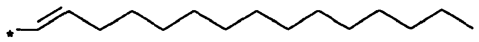

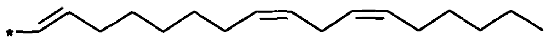

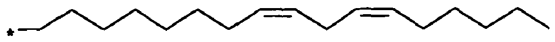

Il est à noter que la composition de l'insaponifiable d'avocat furanique est sensiblement différente de celle de l'insaponifiable d'avocat « classique », dit insaponifiable d'avocat stérolique ou encore huile d'avocat stérolique. En effet, ces insaponifiables d'avocat classiques peuvent contenir des alcanes, des alcools à longue chaîne, des alcool triterpéniques et des stérols (WO99/43298). Ils sont principalement stéroliques.

**Tableau comparatif entre insaponifiables stérolique et furanique d'avocat (tableau 1)**

| **Insaponifiable d'avocat** | **Huile d'avocat stérolique** | **Huile d'avocat furanique** |
|---|---|---|
| Mode d'obtention | Fruit frais par centrifugation | Fruitsséchés à chaud par pression |
| COMPOSITION | en % | en % |
| Fraction stérolique | 40-60 | 3-8 |
| Fraction H (furannes) | 0 | 50-80 |
| Autres (alcools gras, tacophérols, squalène ...) | 60-40 | ≤15 |

Ainsi, la demande internationale WO99/43298, qui enseigne qu'un insaponifiable d'avocat classique peut être utilisé dans le traitement de vergetures, est muette quant à l'utilisation thérapeutique ou cosmétique d'insaponifiables d'avocat furaniques.

Dans l'insaponifiable d'avocat furanique, les 2-alkyles furannes représentent 30% à 70% en poids de cet insaponifiable, par rapport au poids total de l'insaponifiable.

L'huile d'avocat peut contenir 2 à 4 % en poids de 2-alkyles furanes, par rapport au poids total de l'huile.

Selon l'invention, on utilise une fraction furanique purifiée de l'insaponifiable d'avocat, comprenant 70 à 100% en poids, avantageusement 90 % à 100% en poids, de 2-alkyles furannes par rapport au poids total de la fraction, pour la préparation de la composition cosmétique dépigmentante.

L'insaponifiable est la fraction d'un corps gras qui, après action prolongée d'une base alcaline, reste insoluble dans l'eau et peut être extraite par un solvant organique. Cinq grands groupes de substances sont présents dans la plupart des insaponifiables d'huiles végétales : hydrocarbures saturés ou insaturés, alcools aliphatiques ou terpéniques, stérols, tocophérols, les pigments caroténoïdes et xanthophiles.

Les dérivés furaniques de l'huile d'avocat sont des composés connus de l'homme du métier, ils ont par exemple été décrits dans Farines, M. et al, 1995, J. of Am. Oil Chem. Soc. 72,473.

L'insaponifiable d'avocat riche en lipides furaniques a déjà été décrit pour son utilisation dans la fabrication d'un médicament ayant une action bénéfique et curative sur le tissu conjonctif, notamment dans le traitement de pathologies inflammatoires telles que l'arthrose, les parodontites et la sclérodermie.

Dans le cadre de la présente invention, pour l'insaponifiable d'avocat furanique, les expressions « alkyle furanne(s) » et « lipide(s) furanique(s) » sont des expressions synonymes.

L'avocat est avantageusement choisis parmi les variétés Hass, Fuerte, Ettinger, Bacon, Nabal, Anaheim, Lula, Reed, Zutano, Queen, Criola Selva, Mexicana Canta, Region Dschang, Hall, Booth, Peterson, Collinson Red, plus avantageusement, les variétés Hass, Fuerte et Reed. De préférence, on retiendra les variétés Hass, Fuerte, Ettinger et Bacon, et plus avantageusement les variétés Hass et Fuerte.

Dans la fraction furanique purifiée de l'insaponifiable d'avocat, telle que développée par les Laboratoires Expanscience (cf. la demande internationale WO 01/21605), les 2-alkyles furannes représentent de 70 à 100 % en poids, par rapport au poids de la fraction, et les proportions relatives massiques en chacun des furannes identifiés sont données dans le tableau 2 suivant :

**Tableau 2**

| *Composition en 2-alkyles furannes de la fraction furanique purifiée de l'insaponifiable d'avocat* | *% en poids* |
|---|---|
| 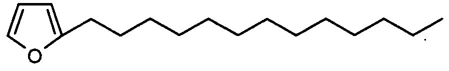 | 3-12 |
| 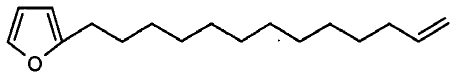 | 1-8 |
| 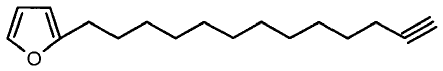 | 1-5 |
| 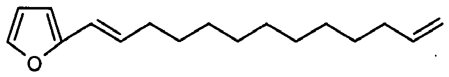 | 1-6 |
| 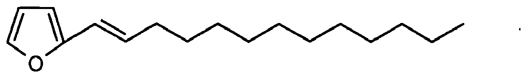 | 5-20 |
| 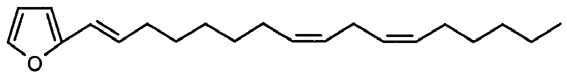 | 10-30 |
| 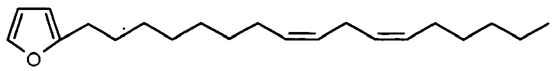 | 35-75 |

Dans la demande internationale WO 01/21605, les laboratoires Expanscience ont mis en oeuvre un procédé spécifique qui permet d'obtenir une extraction sélective des lipides furaniques de l'avocat avec une teneur de plus de 80% en poids de lipides furaniques, voire proche de 100%.

Ce procédé comprend les étapes consistant à préparer un insaponifiable d'avocat, puis à soumettre l'insaponifiable d'avocat à une étape de distillation moléculaire en utilisant des moyens de température et de pression réglés pour obtenir un distillat comprenant principalement des lipides furaniques d'avocat.

De préférence, l'insaponifiable d'avocat est préparé à partir du fruit préalablement traité thermiquement, avant l'extraction de l'huile et la saponification, comme décrit en particulier dans la demande de brevet FR 2 678 632. Ce traitement thermique consiste en un séchage contrôlé du fruit, frais de préférence, pendant au moins quatre heures, avantageusement entre 24 et 48 heures, à une température de préférence d'au moins environ 80°C et de préférence comprise entre environ 80 et environ 120°C ; la température et le temps de séchage étant dépendants l'un de l'autre.

Avant sa saponification, l'huile peut être préalablement enrichie en insaponifiable en séparant une majorité des constituants de l'insaponifiable que l'on récupère dans un concentrat. Différentes méthodes peuvent être utilisées : cristallisation par le froid, extraction liquide-liquide, distillation moléculaire. La distillation moléculaire est particulièrement préférée et elle est réalisée avantageusement à une température comprise entre environ 180 et environ 230°C en maintenant une pression comprise entre 10⁻³ et 10⁻² mmHg.

L'insaponifiable d'avocat obtenu comme décrit ci-dessus est ensuite soumis à une étape de distillation moléculaire. Cette étape de distillation moléculaire est réalisée avec des moyens de température réglés pour une température comprise entre 100 et 160°C et des moyens de pression réglés pour une pression comprise entre 10⁻³ et 5.10⁻² mmHg. En particulier, les moyens de température sont réglés pour une température comprise entre 100 et 140°C et les moyens de pression sont réglés pour une pression comprise entre 10⁻³ et 5.10⁻² mmHg, pour obtenir un distillat comprenant principalement des lipides furaniques d'avocat.

Dans la demande internationale WO 04/016106, les laboratoires Expanscience ont mis au point un procédé permettant d'obtenir avec un rendement élevé, un insaponifiable d'avocat riche en lipides furaniques, à savoir d'une teneur variant de 50 à 80%, présentant de faibles teneurs en produits lourds et en peroxydes.

Ce procédé comprend les étapes successives suivantes :
(1) une étape de déshydratation contrôlée des avocats frais ou ayant subi des transformations préalables, réalisée à une température comprise entre -50°C et 75°C,
(2) une étape d'extraction de l'huile des fruits déshydratés,
(3) une étape, alternativement,
   - a. de traitement thermique de l'huile extraite à une température pouvant varier de 80 à 150°C, éventuellement sous atmosphère inerte, puis d'une étape de concentration de l'huile en sa fraction insaponifiable ou bien,
   - b. d'une étape de concentration de l'huile en sa fraction insaponifiable, puis d'un traitement thermique à une température pouvant varier de 80 à 150°C, éventuellement sous atmosphère inerte, suivie
(4) d'une étape de saponification et d'extraction de l'insaponifiable.

On entend plus généralement par déshydratation, effectuée à l'étape (1) du procédé, l'ensemble des techniques connues de l'homme de métier et qui permettent d'extraire l'eau d'un composé. Parmi ces techniques on préfère le séchage en séchoirs ventilés, en couche mince et sous courant d'air chaud, à une température comprise entre 70 et 75°C, pendant 8 à 36 heures.

L'étape de traitement thermique mise en oeuvre à l'étape (3) a. ou b. peut se faire en présence ou non d'un catalyseur acide, de préférence des alumines acides. L'étape de concentration de l'étape (3)a. ou (3)b. peut être une cristallisation à froid ou une distillation moléculaire.

De façon alternative, la préparation d'une fraction insaponifiable d'avocat composée d'alkyl furannes peut mettre en oeuvre des matières premières qui sont des co-produits issus des procédés d'extraction des huiles d'avocat. Notamment les huiles obtenues à partir des fruits frais, sans séchage préalable des fruits.

Parmi ces co-produits on peut citer de manière non exhaustive : i) les phases grasses et ii) aqueuses issues des procédés dits de centrifugation, ou encore celles issues des procédés dits « enzymatiques », qui comprennent notamment une étape de pré-digestion enzymatique des cellules végétales de la pulpe, afin de faciliter la libération des lipides du fruit. Les résidus solides de centrifugation (culots de centrifugeuse) qui sont extraits du débourbage des huiles brutes peuvent aussi constituer une matière première intéressante.

Aussi, toujours de façon alternative, les pulpes congelées issues de fruits préalablement pelés et dénoyautés peuvent être utilisées. De même, les échappées de désodorisation des huiles d'avocat constituent aussi des sources d'insaponifiables d'avocat et d'alkyl furannes.

En outre, les tourteaux d'avocat; co-produits lors de la pression mécanique à froid des fruits (frais ou séchés) ou de l'extraction liquide-solide de l'huile d'avocat à l'aide d'un solvant peuvent aussi constituer en l'état, une matière première alternative.

Enfin, bien que pauvres en huile, les noyaux d'avocat peuvent potentiellement constituer une source de lipides d'avocat, et notamment d'alkyl furannes.

Dans le cadre de la présente invention, la composition cosmétique dépigmentante comprend avantageusement 0,001 à 25 % en poids de 2-alkyles furannes, plus avantageusement 0,01 à 10% en poids de 2-alkyles furannes, encore plus avantageusement 0,1 à 5% en poids de 2-alkyles furannes, par rapport au poids total de la composition cosmétique et un milieu cosmétiquement acceptable.

Les compositions décrites ici peuvent contenir d'autres actifs cosmétiques à action dépigmentante, conduisant à un effet complémentaire ou synergique. Les 2-alkyles furannes peuvent être associés à des agents dépigmentants connus de l'homme de métier tels que l'hydroquinone et ses dérivés, l'arbutine, l'acide rétinoïque, le rétinol, le rétinaldéhyde, l'acide kojique, l'acide azélaïque, la vitamine B3 ou PP, les dérivés du résorcinol, le résvératrol, des extraits de licorice ou de murier blanc, l'acide alpha-lipoïque, l'acide linoléique, des chélateurs de cations tels que l'EDTA (acide éthylène diamine tétra acétique), les extraits de soja.

Les 2-alkyles furannes peuvent également être associés à des agents cosmétiques anti-oxydants, conduisant à un effet complémentaire ou synergique. Comme exemple d'agents anti-oxydants, on peut notamment citer la vitamine C, la vitamine E, les polyphénols (notamment ceux extraits du thé vert ou de raisin ou de pin), les dérivés soufrés.

Les 2-alkyles furannes peuvent également être associés à des agents cosmétiques dépigmentants tels que le Sepiwhite® (N-undecylenoyl-L-phénylalanine) commercialisé par la société Seppic, conduisant à un effet complémentaire ou synergique.

Selon un autre aspect de l'invention, les compositions cosmétiques selon l'invention contiennent également, éventuellement avec un effet de synergie, au moins un filtre ou un écran solaire UVB et UVA, tels les écrans ou filtres minéraux et/ ou organiques connus de l'homme du métier qui adaptera leur choix et leur concentration en fonction du degré de protection recherché.

Les compositions cosmétiques décrites ici peuvent également contenir des agents exfoliants tels que les alpha- hydroxyacides et salicylique et les dérivés sous forme d'ester par exemple.

Enfin, les compositions cosmétiques décrites ici peuvent également contenir des agents anti-inflammatoires ou apaisants, des agents désensibilisants cutanès tels que des AINS (Anti-inflammatoires non stéroïdiens), des dermocorticoïdes, des agonistes PPAR (peroxysme proliferator activated receptor : récepteur activé par les proliférateurs de peroxysomes),des dérivés de réglisse, de bisabolol, d'isoflavones, (de soja par exemple) glycosilés ou non, du palmitoylèthanolamide, des insaponifiables à base de phytostérols et de vitamines E, des anti-COX et/ ou LOX (inhibiteur de cyclo-oxygènase et/ou de lipoxydase), des eaux thermales, marines ou reconstituées à partir d'oligoéléments exogènes.

La composition cosmétique décrite ici comprend un support cosmétiquement acceptable, c'est à dire un support compatible avec la peau. Elle peut avantageusement se présenter sous toutes les formes galéniques normalement utilisées pour une application topique, notamment sous forme d'une solution aqueuse, hydroalcoolique ou huileuse, d'une émulsion huile- dans-eau ou eau-dans-huile ou multiple, d'un gel aqueux ou huileux, d'un produit anhydre liquide, pâteux ou solide, d'une dispersion d'huile dans une phase aqueuse à l'aide de sphérules (nanosphères, nanocapsules, vésicules lipidiques), d'un dispositif trans-dermique ou sous toute forme pour application topique.

Cette composition peut être plus ou moins fluide et avoir l'aspect d'une crème blanche ou colorée, d'une pommade, d'un lait, d'une lotion, d'un sérum, d'une pâte, d'une

mousse ou d'un gel. Elle peut éventuellement être appliquée sur la peau sous forme d'aérosol. Elle peut également se présenter sous forme solide, et par exemple sous forme de stick. Elle peut aussi être appliquée au moyen d'un patch.

La composition décrite ici peut contenir également les adjuvants habituels dans le domaine cosmétique, dermatologique et/ou pharmacologique, tels que les stabilisants, les conservateurs, les antioxydants, les solvants, les parfums, les agents chélateurs, les absorbeurs d'odeur, des filtres chimiques ou minéraux, des pigments minéraux, les tensioactifs, les polymères, les huiles de silicone et les matières colorantes.

L'invention décrit également l'utilisation de la fraction furanique décrite précedement pour réduire et/ou supprimer les taches de pigmentation, où on applique par voie topique une composition cosmétique comprenant une fraction furanique purifiée de l'insaponifiable d'avocat comprenant des 2-alkyle furannes en tant que principe actif cosmétique dépigmentant. Cette méthode de traitement cométique permet d'uniformiser le teint. La composition cosmétique est avantageusement telle que définie précédemment.

Les tâches de pigmentation peuvent être sans limitation des tâches de vieillesse, des tâches U.V. induites ou des tâches de phototoxicité (parfum, médicament, toxique exogène, brûlure) ou des chloasmas.

L'invention décrit aussi l'utilisation de ladite fraction furanique pour éclaircir la peau, où on applique par voie topique une composition cosmétique comprenant une fraction furanique purifiée de l'insaponifiable d'avocat comprenant des 2-alkyle furannes en tant que principe actif dépigmentant. La composition cosmétique est avantageusement telle que définie précédemment.

Les propriétés dépigmentantes une fraction furanique purifiée de l'insaponifiable d'avocat comprenant des 2-alkyle furannes peuvent, conduire à l'utilisation d'une fraction furanique purifiée de l'insaponifiable d'avocat comprenant des 2-alkyle furannes en tant que principe actif pour la préparation d'un médicament actif comme dépigmentant.

Une fraction furanique purifiée de l'insaponifiable d'avocat comprenant des 2-alkyle furannes utilisée pour la fabrication du médicament est avantageusement telle que définie précédemment. Elle peut être utilisée en association, éventuellement avec un effet de synergie, avec au moins un autre agent dépigmentant tel que défini précédemment et/ou au moins un filtre solaire organique ou minéral et/ou un agent anti-inflammatoire.

Les modes d'administration, les posologies et les formes galéniques optimales des composés et compositions selon l'invention peuvent être déterminés selon les critères généralement pris en compte dans l'établissement d'un traitement cosmétique et/ou dermatologique et/ou pharmaceutique, adapté à un patient comme par exemple le type de peau.

Les exemples suivants illustrent la présente invention.

### Exemple 1 : crème de soin dépigmentant n° 1

| **INGREDIENTS** | **% p/p** |
|---|---|
| Eau | QSP 100 |
| Di-C₁₂₋₁₃ Alkyl Malate | 10,000 |
| Polydécène hydrogéné | 5,000 |
| Amidon d'Oryza Sativa | 4,000 |
| Alcool cétéaryl | 3,200 |
| Glycérine | 3,000 |
| Coco-glycérides hydrogénés | 3,000 |
| Stéarate de sorbitane | 3,000 |
| Undécylénoyl Phénylalanine | 2,000 |
| Glucoside ascorbyle | 2,000 |
| Trométhamine | 1,340 |
| Cérésine | 1,000 |
| Glucoside cétéaryle | 0,800 |
| Parfum | 0,500 |
| Gomme xanthane | 0,400 |
| Cétyl phosphate de potassium | 0,400 |
| Gomme sclerotium | 0,300 |
| Hydroxyméthylglycinate de sodium | 0,200 |
| 2-alkyle furanne | 0,100 |
| Tocophérol | 0,100 |

### Exemple 2 : crème de soin dépigmentant n°2

| **INGREDIENTS** | **% p/p** |
|---|---|
| Eau | QSP 100 |
| Di-C12-13 Alkyl Malate | 10,000 |
| Polydécène hydrogéné | 5,000 |
| Amidon d'Oryza Sativa | 4,000 |
| Alcool cétéaryl | 3,200 |
| Glycérine | 3,000 |
| Coco-glycérides hydrogénés | 3,000 |
| Stéarate de sorbitane | 3,000 |
| Trométhamine | 1,340 |
| Cérésine | 1,000 |
| Glucoside cétéaryle | 0,800 |
| Parfum | 0,500 |
| Gomme xanthane | 0,400 |
| Cétyl phosphate de potassium | 0,400 |
| Gomme sclerotium | 0,300 |
| Hydroxyméthylglycinate de sodium | 0,200 |
| 2-alkyle furanne | 0,200 |

### Exemple 3 : Spray dépigmentant SPF (Suncreen Protection Factor : Facteur de protection écran solaire) 30

| **Ingrédients** | **% p/p** |
|---|---|
| Eau | QSP 100 |
| Tétraoctanoate de Pentaérythrityle | 15 à 30 |
| Dioxyde de titane | 1 à 10 |
| Cyclométhicone | 1 à 10 |
| Oxyde de zinc | 1 à 10 |
| Méthylène Bis-Benzotriazolyl tétraméthylbutylphénol | 1 à 5 |
| Benzoate de (C₁₂-C₁₅)alkyle | 1 à 10 |
| 4,5,7-Trihydroxyisoflavone | 0,01 à 10 |
| Glycérine | 1 à 10 |
| Ether de dicaprylyle | 1 à 10 |
| Cyclopentasiloxane | 1 à 10 |
| Ethylhexyl diméthicone éthoxy glucoside | 1 à 10 |
| Propylène Glycol Dioctanoate | 1 à 10 |
| Chlorure de sodium | 1 à 5 |
| Copolymère PEG-45/Dodécyl Glycol | 1 à 5 |
| PEG-30 Dipolyhydroxystéarate | 1 à 5 |
| Huile d'insaponifiable de soja | 1 à 5 |
| Palmitate de dextrine | 1 à 5 |
| 2-alkyle furanne | 0,01 à 3 |
| Extrait *d'aloé barbadensis* | 0,2 |
| Conservateurs | QS |
| Gluconate de zinc | 0,08 |

### Exemple 4 : Crème dépigmentante SPF 50

| **Ingrédients** | % **en poids** |
|---|---|
| Eau | QSP 100 |
| Tétraoctanoate de Pentaérythrityle | 15 à 30 |
| Dioxyde de titane | 1 à 10 |
| Cyclométhicone | 1 à 10 |
| Oxyde de zinc | 1 à 10 |
| Benzoate de (C₁₂-C₁₅) alkyle | 1 à 10 |
| Undécylénoyl Phénylalanine | 0,5 à 2 |
| 2-alkyle furanne | 0,01 à 10 |
| 4,5,7-Trihydroxyisoflavone | 0,01 à 10 |
| Glycérine | 1 à 10 |
| Ether de dicaprylyle | 1 à 10 |
| Cyclopentasiloxane | 1 à 10 |
| Ethylhexyl diméthicone Ethoxy Glucoside | 1 à10 |
| Propylène Glycol Dioctanoate | 1 à 10 |
| Chlorure de sodium | 1 à 5 |
| Copolymère PEG-45/Dodécyl Glycol | 1 à 5 |
| PEG-30 Dipolyhydroxystéarate | 1 à 5 |
| Huile d'insaponifiable de soja | 1 à 5 |
| Palmitate de dextrine | 1 à 5 |
| Conservateurs | QS |
| Extrait *d'aloé barbadensis* | 0,2 |
| Gluconate de zinc | 0,08 |

### Exemple 5 : Crème dépigmentante n°3

| **INGREDIENTS** | **% p/p** |
|---|---|
| Eau | QSP 100 |
| Di-C₁₂₋₁₃ Alkyl Malate | 10,000 |
| Polydécène hydrogéné | 5,000 |
| Amidon d'Oryza Sativa | 4,000 |
| Alcool cétéaryl | 3,200 |
| Glycérine | 3,000 |
| Coco-glycérides hydrogénés | 3,000 |
| Stéarate de sorbitane | 3,000 |
| Glucoside ascorbyle | 2,000 |
| Trométhamine | 1,340 |
| Cérésine | 1,000 |
| Glucoside cétéaryle | 0,800 |
| Parfum | 0,500 |
| Gomme xanthane | 0,400 |
| Cétyl phosphate de potassium | 0,400 |
| Bisabolol | 0,300 |
| Gomme sclérotium | 0,300 |
| Hydroxyméthylglycinate de sodium | 0,200 |
| 2-alkyle furanne | 0,100 |
| Tocophérol | 0,100 |

## Revendications

1. Utilisation cosmétique d'une fraction furanique purifiée de l'insaponifiable d'avocat comprenant, par rapport au poids total de la fraction, 70 à 100% en poids de 2-alkyle furanne répondant à la formule générale (I) 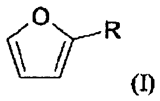 dans laquelle R représente un radical alkyle en C₁₀-C₂₂, un radical alcényle en C₁₀-C₂₂, ou un radical alcynyle en C₁₀-C₂₂, lesdits radicaux alkyles, alcényles et alcynyles pouvant être substitués par un ou plusieurs halogène(s) et/ou par un ou plusieurs fonctions choisies dans le groupe constitué par les fonctions époxyde, hydroxyle (-OH), thiol (-SH), éther (-OR₁), amine primaire (-NH₂), amine secondaire (-NHR₁), amine tertiaire (-NR₁R₂), aldéhyde (-CHO), cétone (-COR₁), acétyle (-O-CO-R₁), avec R₁ et R₂ représentant, indépendamment l'un de l'autre, un atome d'hydrogène, un radical alkyle en C₁-C₃₅ ou un radical alcényle en C₁-C₃₅, en tant que principe actif cosmétique dépigmentant, dans une composition cosmétique dépigmentante.

2. Utilisation cosmétique selon la revendication 1, **caractérisée en ce que** le 2-alkyle furanne répond à la formule générale (I) dans laquelle R représente un radical choisi dans le groupe constitué par les radicaux suivants (*-R) :

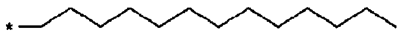

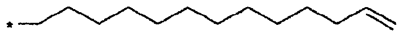

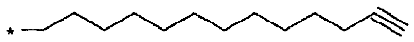

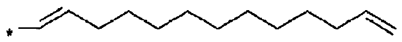

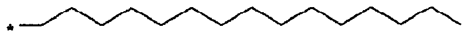

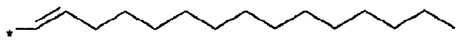

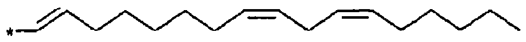

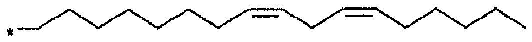

3. Utilisation cosmétique selon l'une des revendications 1-2, **caractérisée en ce que** la composition cosmétique est destinée à améliorer l'uniformité et la clarté du teint.

4. Utilisation cosmétique selon l'une des revendications 1-2 **caractérisée en ce que** la composition cosmétique est destinée à éclaircir la peau.

5. Utilisation cosmétique selon l'une des revendications 1-2, **caractérisée en ce que** la composition cosmétique est destinée à lutter contre les taches de pigmentation.

6. Utilisation selon l'une quelconque des revendications précédentes, **caractérisée en ce que** la composition cosmétique comprend en outre au moins un filtre solaire organique ou minéral.

7. Utilisation selon l'une quelconque des revendications 1-2, **caractérisée en ce que** la composition cosmétique est appliquée par voie topique et est destinée à lutter contre les tâches de pigmentation.

8. Utilisation cosmétique selon la revendication 7, **caractérisée en ce que** les taches de pigmentation sont des tâches de vieillesse, ou des tâches solaires, ou des tâches liées à une phototoxicité ou des chloasmas

## Claims

1. Cosmetic use of a purified furanic unsaponifiable fraction of avocado that contains, compared to the total weight of the fraction, 70% to 100% by weight of the 2-alkyl furan of general formula (I) 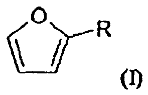 wherein R represents a C₁₀-C₂₂ alkyl radical, a C₁₀-C₂₂ alkenyl radical or a C₁₀-C₂₂ alkynyl radical, said alkyl, alkenyl and alkynyl radicals possibly being substituted by one or more halogens and/or one or more functional groups chosen from the group comprising epoxide, hydroxyl (-OH), thiol (-SH), ether (-OR₁), primary amino (-NH₂), secondary amino (-NHR₁), tertiary amino (-NR₁R₂), aldehyde (-CHO), ketone (-COR₁) and acetyl (-O-CO-R₁) functional groups, with R₁ and R₂ representing, independently of each other, a hydrogen atom, a C₁-C₃₅ alkyl radical or a C₁-C₃₅ alkenyl radical, as depigmenting cosmetic active principle, in a depigmenting cosmetic composition.

2. Cosmetic use according to claim 1, **characterised in that** the 2-alkyl furan is of general formula (I), wherein R represents a radical chosen among the group comprised of the following radicals (*-R) :

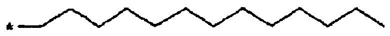

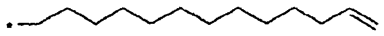

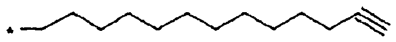

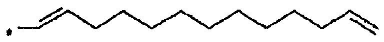

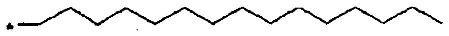

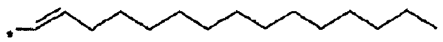

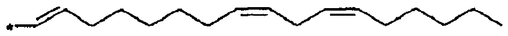

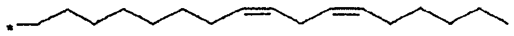

3. Cosmetic use according to claim 1 or claim 2, **characterised in that** the cosmetic composition is intended to improve skin tone uniformity and lightness.

4. Cosmetic use according to claim 1 or claim 2, **characterised in that** the cosmetic composition is intended to lighten the skin.

5. Cosmetic use according to claim 1 or claim 2, **characterised in that** the cosmetic composition is intended act on pigment spots.

6. Use according to any one of the preceding claims, **characterised in that** the cosmetic composition further comprises at least one organic or mineral sun filter.

7. Use according to claim 1 or claim 2, **characterised in that** the cosmetic composition is applied by topical route and is intended to act on pigment spots.

8. Cosmetic use according to claim 7, **characterised in that** the pigment spots are age spots, sun-induced spots, or spots related to phototoxicity or chloasmas.

## Patentansprüche

1. Kosmetische Verwendung einer gereinigten Furanfraktion des Unverseifbaren der Avocado, umfassend, bezogen auf das Gesamtgewicht der Fraktion, 70-100 Gew.% 2-Alkylfuran gemäß der allgemeinen Formel (I) 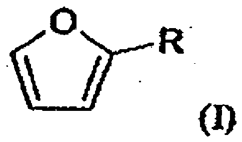 wobei R ein Alkylradikal mit C₁₀ bis C₂₂, ein Alkenylradikal mit C₁₀ bis C₂₂ oder ein Alkinylradikal mit C₁₀ bis C₂₂ repräsentiert, wobei die Alkyl-, Alkenyl- oder Alkinylradikale mit einem oder mehreren Halogenen und/oder mit einer oder mehreren Funktionen substituiert sein können, welchletztere ausgewählt sind aus der Gruppe bestehend aus den Funktionen Epoxid, Hydroxyl (-OH), Thiol (-SH), Ether (-OR₁), primäres Amin (-NH₂), sekundäres Amin (-NHR₁), tertiäres Amin (-NR₁R₂), Aldehyd (-CHO), Keton (-COR₁), Acetyl (-O-CO-R₁), wobei R₁ und R₂ unabhängig voneinander ein Wasserstoffatom, ein Alkylradikal mit C₁ bis C₃₅ oder ein Alkenylradikal mit C₁ bis C₃₅ darstellen, als depigmentierender kosmetischer Wirkstoff in einer depigmentierenden kosmetischen Zusammensetzung.

2. Kosmetische Verwendung nach Anspruch 1, **dadurch gekennzeichnet, dass** das 2-Alkylfuran der allgemeinen Formel (I) entspricht, wobei R ein Radikal repräsentiert, das ausgewählt ist aus der Gruppe bestehend aus den folgenden Radikalen (*-R):

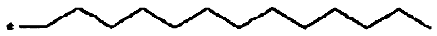

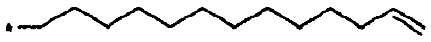

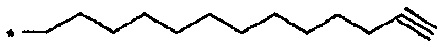

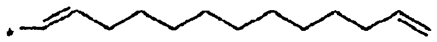

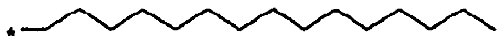

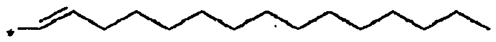

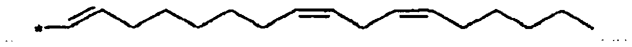

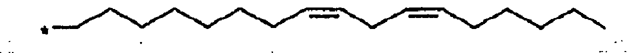

3. Kosmetische Verwendung nach einem der Ansprüche 1 bis 2,
**dadurch gekennzeichnet, dass** die kosmetische Zusammensetzung dazu bestimmt ist, die Einheitlichkeit und die Klarheit des Teints zu verbessern.

4. Kosmetische Verwendung nach einem der Ansprüche 1 bis 2,
**dadurch gekennzeichnet, dass** die kosmetische Zusammensetzung dazu bestimmt ist, die Haut aufzuhellen.

5. Kosmetische Zusammensetzung nach einem der Ansprüche 1 bis 2, **dadurch gekennzeichnet, dass** die kosmetische Zusammensetzung dazu bestimmt ist, Pigmentflecken zu bekämpfen.

6. Verwendung nach einem der vorhergehenden Ansprüche,
**dadurch gekennzeichnet, dass** die kosmetische Zusammensetzung desweiteren mindestens einen organischen oder mineralischen Sonnenfilter umfasst.

7. Verwendung nach einem der Ansprüche 1 bis 2, **dadurch gekennzeichnet, dass** die kosmetische Zusammensetzung auf topischem Wege angewendet wird und dazu bestimmt ist, Pigmentflecken zu bekämpfen.

8. Verwendung nach Anspruch 7, **dadurch gekennzeichnet, dass** die Pigmentflecken Altersflecken, Sonnenflecken oder mit einer Phototoxizität oder Chloasmen zusammenhängende Flecken sind.
